# FASCICULE DE BREVET EUROPEEN

(11) **EP 0 796 268 B1**
(45) Date de publication et mention de la délivrance du brevet: **29.03.2000**
(21) Numéro de dépôt: 95941781.7
(22) Date de dépôt: 06.12.1995
(51) Int. Cl.: C07H 1/06, B01J 8/12, B01J 8/16

(54) **PROCEDE DE PREPARATION EN CONTINU D'UNE SOLUTION DE CETOSE PAR ISOMERISATION D'ALDOSE, ET INSTALLATION DE MISE EN OEUVRE**
KONTINUIERLICHES VERFAHREN ZUR HERSTELLUNG EINER WÄSSRIGEN KETOSELÖSUNG DURCH ISOMERISIERUNG EINER ALDOSE UND ANLAGE ZUR DURCHFÜHRUNG DES VERFAHRENS
METHOD FOR CONTINUOUSLY PREPARING A KETOSE SOLUTION BY ISOMERISING ALDOSE, AND APPARATUS THEREFOR

(30) Priorité: 07.12.1994 FR 9414960
(43) Date de publication de la demande: 24.09.1997
(73) Titulaire: AGRICHIMIE, F-97231 Robert (FR)
(72) Inventeur: DURAND, Germain-Robert, F-34830 Clapiers (FR); FAUGERAS, Pierre, F-30130 Pont-Saint-Esprit (FR); LAPORTE, Françoise, F-30165 Saint-Laurent-des-Arbres (FR); MOREAU, Claude, F-34880 Laverune (FR); NEAU, Marie-Claude, F-31290 Gardouch (FR); ROUX, Alain, F-30100 Arles (FR); ROUX, Gabriel, F-38240 Meylan (FR); TICHIT, Didier, Résidence Le Vallon-des-Sources, F-34090 Montpellier (FR)
(74) Mandataire: Cabinet BARRE LAFORGUE & associés
(86) Numéro de dépôt international: FR9501616
(87) Numéro de publication internationale: WO9617858

(56) Documents cités:
- WO-A-92/10486
- US-A- 4 014 711
- US-A- 4 373 025
- US-E- R E33 105
- DATABASE WPI Derwent Publications Ltd., London, GB; AN 76-74905X[40] XP002001398 & JP,A,51 095 143 (TORAY) , 21 Août 1976

## Description

L'invention concerne un procédé et une installation de préparation en continu d'une solution de cétose -notamment de cétohexose tel que le fructose- à partir d'une solution sucrée de départ comprenant au moins un aldose -notamment un aldohexose tel que le glucose-.

Les solutions sucrées extraites des matières naturelles (chicorée, betterave, canne à sucre, blé...) contiennent des osides qui, par hydrolyse, produisent des solutions sucrées mixtes de cétoses, d'aldoses et d'osides. Les aldoses, et notamment les aldohexoses tels que le glucose, sont des sucres qui peuvent être obtenus à moindre coût compte tenu de leur présence en grande quantité dans les produits naturels. Au contraire, les cétoses, plus rares dans les produits naturels, sont de coût plus élevé et sont difficiles à extraire ou à obtenir avec une grande pureté. Or, les cétoses, et notamment les cétohexoses tels que le fructose, possèdent des propriétés spécifiques intéressantes. En particulier, ils ont un pouvoir édulcorant différent et sont des précurseurs de nombreux dérivés aux propriétés intéressantes, par exemple le HMF. Mais tant pour l'industrie agro-alimentaire, que pour l'industrie de la chimie organique, l'utilisation des cétoses suppose de disposer à moindre coût d'une solution d'une grande pureté, notamment exempte d'aldose dont les propriétés gustatives et physico-chimiques sont différentes de celles des cétoses.

Par exemple, le prix d'obtention du glucose est classiquement de l'ordre de 2 à 4 fois celui du saccharose (oside d'origine naturelle) alors que celui du fructose est classiquement de l'ordre de 5 à 8 fois celui du saccharose, soit de l'ordre de 2 à 4 fois celui du glucose.

On a déjà pensé à isomérer les aldoses en cétoses et notamment le glucose en fructose. Cette réaction d'isomérisation est un équilibre difficile à déplacer et qui ne permet pas d'obtenir une pureté de 100 % de cétose par voie chimique. Il est donc nécessaire d'avoir recours à une étape d'extraction ultérieure qui, elle-même, n'aboutit pas à une pureté parfaite de 100 % en cétose à un coût raisonnable (cf US-A-4.373.025).

L'isomérisation chimique du glucose en fructose en milieu homogène peut être effectuée en présence d'une catalyse basique. Néanmoins, plusieurs autres isomères ainsi que des produits de décomposition se forment simultanément (tels que le mannose, le sorbose, le psicose, le tagatose...).

L'isomérisation du glucose en fructose en présence d'une résine échangeuse d'ions a aussi été envisagée. Néanmoins, le fructose formé diffuse dans la résine et se dégrade plus rapidement que le glucose. Dès lors, les procédés connus conduisent à des purifications difficiles dues à la présence de produits colorés, d'odeurs, de cendres...

On a aussi envisagé d'effectuer l'isomérisation en présence d'une enzyme. Néanmoins, les inconvénients de ce type de réaction résident dans le prix de production de l'enzyme et dans la sensibilité aux paramètres extérieurs (Ph, température...). En outre, l'isomérisation enzymatique conduit (à partir de glucose pur) à des taux de fructose nécessitant une séparation ultérieure.

JP - 51095143 décrit l'isomérisation d'une solution de glucose à 50 Brix en présence d'un aluminosilicate sous forme alcaline dans une colonne à lit fixe. Le rendement en fructose est de l'ordre de 40% et une étape ultérieure de séparation est nécessaire. Ce procédé présente aussi les inconvénients sus-mentionnés et n'a donc pa été exploité industriellement.

L'invention vise donc à pallier ces inconvénients en proposant un procédé de préparation en continu d'une solution pure de cétose, notamment de cétohexose et en particulier de fructose, applicable à l'échelle industrielle à moindre coût, et notamment à un coût comparable à celui des aldoses, notamment des aldohexoses, et en particulier du glucose.

L'invention vise en particulier à proposer un procédé économique de préparation en continu d'une solution pure de cétose, à partir d'une solution d'aldose ou d'un mélange d'aldose(s) et de cétose(s).

En particulier, l'invention vise à proposer un procédé de préparation d'une solution pure de fructose à partir d'une solution de glucose, ou de fructose et de glucose, et ce à un coût plus faible que dans l'art antérieur, et notamment compatible avec une rentabilité industrielle.

L'invention vise aussi à proposer un tel procédé effectué en continu en une seule étape et qui peut être mis en oeuvre industriellement dans des conditions rentables dans une installation de taille et de capacité réduites à faibles prix de revient et coûts de fonctionnement.

L'invention vise en outre à proposer une installation de mise en oeuvre du procédé.

Pour ce faire, l'invention concerne un procédé de préparation en continu d'une solution de cétose -notamment de cétohexose- à partir d'une solution sucrée de départ comprenant au moins un aldose -notamment au moins un aldohexose-, caractérisé en ce qu'on utilise au moins un réacteur multicontact continu vertical, en ce qu'on introduit en continu la solution sucrée de départ dans le réacteur, en ce qu'on fait circuler en continu dans le réacteur au moins un tectosilicate ou une argile apte à former un catalyseur hétérogène d'isomérisation des aldoses en cétoses, et en ce qu'on fait circuler de haut en bas dans le réacteur au moins un solide microporeux d'adsorption sélective choisi pour adsorber sélectivement soit les aldoses de la solution de départ, soit les cétoses issus de l'isomérisation, et être compatible avec les conditions d'isomérisation.

Dans toute la présente demande, l'expression "solide microporeux" désigne de façon générale tout composé minéral d'origine naturelle ou synthétique formé par des enchaînements tridimensionnels de tétraèdres T0₄ avec T représentant au moins deux éléments différents de la classification périodique tels que Si, Al, B, Fe, Ga, Ge, etc... Dans cet enchaînement, les atomes d'oxygène des tétraèdres T0₄ sont en commun avec les tétraèdres voisins.

Un solide microporeux est un solide présentant des pores de dimensions généralement inférieures à environ 10⁻⁹ m. Il est à noter qu'un solide microporeux se distingue des solides macroporeux (dont les pores ont des dimensions généralement supérieures à 10⁻⁸ m) tels que les résines, et des solides mésoporeux (dont les pores ont des dimensions généralement comprises entre 2.10⁻⁹ m et 10⁻⁸ m).

Si on utilise un solide d'adsorption sélective des cétoses, une étape de désorption ultérieure est prévue. Une telle étape est néanmoins simple, peu coûteuse et fournit les cétoses avec une grande pureté.

Si on utilise un solide microporeux apte à adsorber sélectivement les aldoses, on récupère le cétose isomérisé dans un solvant, notamment un solvant à contre-courant circulant de bas en haut qui peut être le même que celui de la solution de départ, à savoir de l'eau.

L'invention concerne donc aussi un procédé caractérisé en ce que :
- on utilise un seul réacteur multicontact,
- on introduit la solution sucrée de départ à un niveau intermédiaire du réacteur,
- on choisit au moins un solide microporeux d'adsorption sélective des aldoses de la solution sucrée de départ,
- on introduit dans le réacteur un courant continu d'un solvant de la solution sucrée de départ à un niveau du réacteur inférieur à celui auquel on introduit la solution sucrée de départ,
- on extrait en continu, à contre-courant, la solution de cétose à un niveau du réacteur au-dessus de celui auquel on introduit la solution sucrée de départ, de sorte que le procédé peut être effectué en continu en une seule étape dans un seul réacteur.

Dans un procédé selon l'invention, on effectue l'isomérisation dans une zone différente, -notamment située en dessous- d'une zone de séparation, notamment d'une zone d'adsorption des aldoses.

Selon l'invention, on contrôle la température au sein du(des) réacteur(s) selon au moins deux zones distinctes dont la première est maintenue à une température comprise entre 30° C et 90° C choisie pour favoriser l'adsorption par le solide microporeux, et dont la deuxième est maintenue à une température comprise entre 90° C et 110° C choisie pour favoriser l'isomérisation des aldoses en cétoses. Avantageusement et selon l'invention, on maintient une température de l'ordre de 60° C dans la première zone d'adsorption et une température de l'ordre de 95° C à 100° C dans la deuxième zone d'isomérisation.

Selon l'invention, la première zone d'adsorption est située au-dessus de l'introduction de la solution sucrée de départ dans le réacteur, et la deuxième zone d'isomérisation est située en-dessous de l'introduction de la solution sucrée de départ dans le réacteur. Ainsi, on maintient des températures différentes au-dessus et en dessous de l'introduction de la solution de départ dans le réacteur, à savoir une température comprise entre 30° C et 90° C, notamment de l'ordre de 60° C correspondant à la première zone d'adsorption des aldoses par le solide microporeux au-dessus de l'introduction de la solution de départ, et une température comprise entre 90° C et 110° C, notamment de l'ordre de 95° C, correspondant à une deuxième zone d'isomérisation en dessous de l'introduction de la solution de départ.

L'introduction d'un solvant (eau) en partie inférieure permet de laver le solide microporeux et de capter les cétoses formés par isomérisation.

Les tectosilicates sont des composés microporeux caractérisés par une structure comportant :
- une charpente tridimensionnelle formée par l'enchaînement de tétraèdres TO4, SiO4, T représentant un élément de la classification tel que Al, B, Ga, Ge,... et,
- un réseau monodimensionnel, bidimensionnel, ou tridimensionnel de canaux et cavités de dimensions moléculaires contenant des cations de compensation éventuels, de l'eau ou d'autres molécules ou sels.

Parmi les tectosilicates utilisables dans le cadre de l'invention, on peut citer notamment les zéolithes (aluminosilicates). On peut aussi néanmoins utiliser des borosilicates ou des aluminophosphates, ou des silicoaluminophosphates...

Un tectosilicate fonctionnalisé par des cations métalliques est un solide microporeux qui présente des sites métalliques alcalins catalytiques au sein de sa structure.

Egalement, selon l'invention, on utilise, à titre de catalyseur hétérogène d'isomérisation, un tectosilicate ou une argile, notamment sous forme non protonique en particulier fonctionnalisé par des cations métalliques -notamment alcalins ou alcalino-terreux-. Le tectosilicate ou argile sous forme cationique (non protonique) est choisi pour présenter une basicité suffisante pour catalyser l'isomérisation.

Egalement, selon l'invention, on utilise un tectosilicate ou une argile à titre de solide microporeux d'adsorption sélective. En outre, selon l'invention, on utilise un solide microporeux d'adsorption sélective en morceaux préformés, notamment sous forme extrudée.

Dans une première variante de l'invention, on choisit et on utilise un catalyseur distinct du solide microporeux d'adsorption sélective.

Dans une deuxième variante de l'invention, on choisit et on utilise un tectosilicate ou une argile apte à faire office simultanément de catalyseur d'isomérisation et de solide microporeux d'adsorption sélective.

Dans ce cas, selon l'invention, on choisit un tectosilicate ou une argile qui fait office de catalyseur dans une plage de températures différente de celle où il fait office de solide microporeux d'adsorption sélective des aldoses. Plus particulièrement, on choisit un tectosilicate ou une argile présentant une adsorption sélective des aldoses à une température distincte de celle où l'effet de catalyse de l'isomérisation par ce solide est optimale.

On sait définir les caractéristiques d'un solide microporeux pour qu'il présente une adsorption sélective à l'égard soit des aldoses (notamment du glucose), soit des cétoses (notamment du fructose). Selon l'invention, on utilise une zéolithe à titre de catalyseur et/ou de solide d'adsorption sélective.

Selon l'invention, à titre de solide d'adsorption sélective des aldoses, notamment du glucose, on utilise une zéolithe X sous forme potassium ou une zéolithe X sous forme baryum. Ces zéolithes présentant en outre une bonne basicité peuvent être utilisées seules dans un procédé selon l'invention pour faire office à la fois de catalyseur d'isomérisation et d'adsorbant sélectif des aldoses comme indiqué ci-dessus.

A titre de solide d'adsorption sélective des cétoses, notamment du fructose, on peut utiliser une zéolithe X sous forme sodium, ou une zéolithe Y sous forme potassium, ou une zéolithe X sous forme strontium ou une zéolithe Y sous forme calcium.

Avantageusement, le procédé selon l'invention est mis en oeuvre dans une colonne pulsée.

Les colonnes pulsées sont des dispositifs connus multicontacts verticaux d'extraction ou de séparation dans lesquels on peut entretenir des pulsations (cf par exemple le document "Pulsed Perforated-Plate Columns", D.H. Logsdail, M.J. Slaten, Handbook of Solvent Extraction, Teh C.Lo Malcolm H.I. Baird, Carl Hanson, Krieiger Publishing Company, Malabar Florida, 1991, 11-2, p 335-372, incorporé par référence à la présente description).

Il est à noter que dans un procédé selon l'invention, la colonne pulsée fait office non pas uniquement de séparateur, mais également et surtout de réacteur multicontact continu. On réalise ainsi simultanément dans la colonne pulsée, en une seule étape, la réaction et l'extraction sélective en continu du produit de réaction.

L'invention concerne aussi une installation de mise en oeuvre d'un procédé selon l'invention, caractérisée en ce qu'elle comporte un réacteur continu vertical multicontact, notamment une colonne pulsée, et des moyens de contrôle de la température régnant au sein du réacteur selon au moins deux zones distinctes correspondant respectivement à au moins deux températures distinctes.

L'installation selon l'invention est aussi caractérisée en ce qu'elle comporte une alimentation en solution de départ située à un niveau intermédiaire du réacteur, et en ce que les moyens de contrôle sont adaptés pour contrôler indépendamment la température au-dessus et en dessous de l'alimentation en solution de départ.

L'invention concerne en outre un procédé et une installation comprenant en combinaison tout ou partie des caractéristiques mentionnées ci-dessus ou ci-après.

L'invention permet l'obtention en continu en une seule étape de cétoses par isomérisation d'aldoses.

Il est à noter à cet égard que les caractéristiques combinées de l'invention permettent l'obtention de ce résultat par le fait qu'il est possible d'opérer avec des sirops concentrés (Brix supérieur à 65) à haute température, avec une faible durée de réaction, les conditions réactionnelles (notamment l'absorption simultanée) déplaçant l'équilibre fortement.

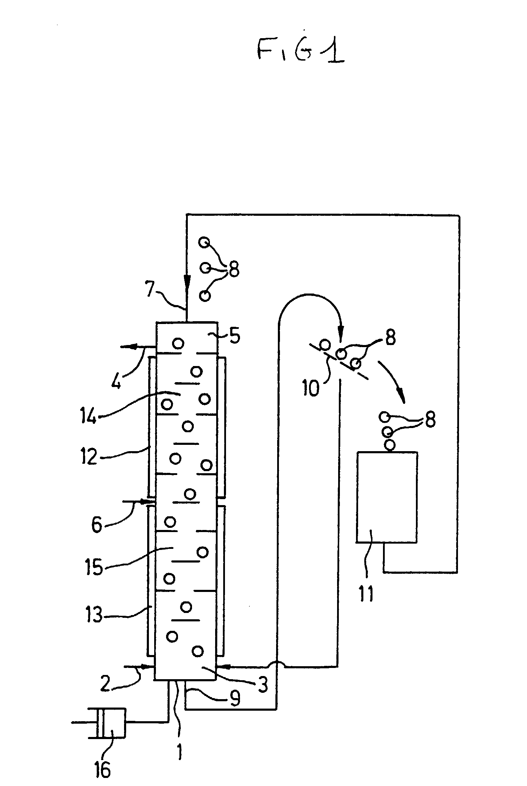

D'autres caractéristiques et avantages de l'invention apparaîtront à la lumière de la description suivante qui se réfère à la figure unique annexée qui est un schéma d'une installation de mise en oeuvre d'un procédé selon l'invention de préparation en continu en une seule étape d'une solution pure de cétose.

L'installation selon l'invention représentée sur la figure comporte une colonne pulsée 1 qui fait office de dispositif vertical multicontact continu de réaction/extraction. Cette colonne pulsée 1 comprend une alimentation 2 en partie inférieure 3 par laquelle on peut introduire un liquide, notamment de l'eau, et une sortie 4 à sa partie supérieure 5 par laquelle la solution de cétose pure préparée est extraite.

En outre, la colonne pulsée 1 est dotée d'une alimentation 6 en solution sucrée de départ, située à un niveau intermédiaire, c'est-à-dire entre l'alimentation 2 inférieure en eau et l'extraction 4 supérieure de la solution de cétose. L'alimentation 6 en solution de départ est située à un niveau au moins sensiblement médian dans l'exemple représenté. Selon les cinétiques respectives de l'adsorption et de l'isomérisation, l'alimentation 2 peut être déplacée vers le haut ou vers le bas par rapport à l'exemple représenté schématiquement sur la figure.

La colonne pulsée 1 est également dotée d'une introduction 7 supérieure de composé(s) solide(s) en morceaux 8. Ces morceaux 8 de composé(s) solide(s) circulent de haut en bas et sont donc récupérés à la partie inférieure 3 de la colonne pulsée 1 par une sortie 9.

Dans le cas où on utilise une zéolithe X sous forme potassium ou sous forme baryum préformée (extrudée en billes, bâtonnets, cylindres, ...), cette zéolithe fait office simultanément de catalyseur d'isomérisation et de solide microporeux d'adsorption sélective des aldoses, et notamment du glucose. Une telle zéolithe forme en effet un tamis moléculaire adsorbant préférentiellement le glucose par rapport au fructose. Ces morceaux circulent donc de haut en bas à travers la colonne pulsée 1, et à contre-courant de la phase liquide qui circule de bas en haut.

En variante non représentée, on pourrait utiliser un catalyseur d'isomérisation distinct du composé solide d'adsorption sélective. Ce catalyseur peut être introduit en simultané avec les morceaux de composé solide d'adsorption à la partie supérieure 5 de la colonne 1, ou au contraire être mélangé sous forme pulvérulente en suspension dans la solution sucrée de départ, grâce à un mélangeur situé en amont de l'alimentation 6 qui est alors déplacée en partie inférieure 3 de la colonne pulsée 1. Dans ce dernier cas, un filtre est prévu à la sortie de l'extraction 4 de la solution de cétose pour séparer le catalyseur en suspension dans cette solution. Egalement, dans cette variante, le catalyseur ne doit pas faire office de composé d'adsorption sélective de l'un ou l'autre des sucres.

Les morceaux 8 de composé(s) solide(s) récupérés à la partie inférieure 3 de la colonne pulsée 1 sont amenés à un filtre 10, grâce à un dispositif d'ascenseur hydraulique. A la sortie du filtre 10, les morceaux 8 peuvent être récupérés par un traitement effectué en 11 consistant à désorber les aldoses et à refonctionnaliser le composé solide pour le rendre à nouveau apte à remplir les fonctions auxquelles on le désigne. Par exemple, dans le cas d'une zéolithe X sous forme potassium, celle-ci peut être calcinée pour désorber les aldoses, puis refonctionnalisée sous forme potassium. A la sortie du traitement 11, les morceaux 8 de composé(s) solide(s) peuvent être recyclés à la partie supérieure 5 de la colonne pulsée 1.

En outre, l'installation comporte des moyens 12, 13 de contrôle de la température régnant au sein de la colonne pulsée 1 selon au moins deux zones 14, 15 distinctes correspondant respectivement à au moins deux températures distinctes. Ces moyens 12, 13 de contrôle de températures sont aptes à contrôler indépendamment la température au-dessus (dans la zone supérieure 14) et en dessous (dans la zone inférieure 15) de l'alimentation 6 en solution de départ.

Dans l'exemple représenté, la colonne pulsée 1 est dotée de deux doubles enveloppes 12, 13, à savoir une double enveloppe supérieure 12 disposée au-dessus de l'alimentation 6 en solution de départ, et une double enveloppe 13 disposée en dessous de l'alimentation 6 de solution de départ. Un dispositif d'alimentation et de contrôle du fonctionnement des doubles enveloppes 12, 13 est prévu de façon connue en soi et non représentée sur la figure. La zone 14 supérieure du réacteur située en regard de la double enveloppe supérieure 12 peut ainsi être maintenue à une température différente de la zone 15 inférieure en regard de la double enveloppe inférieure 13. Et on peut contrôler indépendamment l'une de l'autre les. températures régnant respectivement dans chacune de ces zones 14, 15.

Dans le procédé selon l'invention, la zone supérieure 14 constitue une première zone d'adsorption dans laquelle on maintient une température comprise entre 30° C et 90° C, notamment de l'ordre de 60° C, choisie pour favoriser l'adsorption par le solide microporeux, notamment l'adsorption sélective des aldoses par la zéolithe X sous forme potassium ou baryum. La zone inférieure 15 constitue une deuxième zone d'isomérisation dans laquelle on maintient une température comprise entre 90° C et 110° C, notamment de l'ordre de 95° C, pour favoriser l'isomérisation des aldoses en cétoses en présence du catalyseur d'isomérisation.

Ainsi, dans la zone supérieure 14, les morceaux 8 de zéolithe absorbante captent sélectivement les aldoses, notamment le glucose, en solution dans la phase liquide et les transportent jusqu'à la zone inférieure 15 d'isomérisation. Dans cette zone inférieure, les aldoses adsorbés sur la zéolithe sont isomérisés par les sites cationiques, et le courant d'eau capte les cétoses formés, notamment le fructose qui est entraîné, avec le fructose issu éventuellement de l'alimentation 6 de la solution de départ, jusqu'à la sortie 4 supérieure.

Enfin, la colonne pulsée 1 est dotée d'un dispositif 16 apte à entretenir des pulsations des phases liquides, de façon connue en soi.

L'amplitude et la fréquence des pulsations sont déterminées pour assurer les circulations appropriées sus-décrites des phases liquides et solides. Il en va de même du garnissage de la colonne pulsée qui peut être du type paniers et couronnes ou disques et couronnes.

### EXEMPLE 1

On étudie la réaction d'isomérisation d'une solution de départ de glucose en fructose avec une zéolithe X sous forme potassium.

Dans un réacteur de 500 ml discontinu agité, on réalise un sirop de glucose en introduisant 170 g de glucose avec de l'eau. On chauffe le réacteur à 95° C tout en agitant la solution jusqu'à la dissolution complète du glucose.

On ajoute ensuite 102 g de zéolithe X sous forme potassium préalablement imprégnée du restant d'eau à 95° C.

La zéolithe utilisée présente une coefficient de partage d'adsorption du fructose par rapport au glucose tel qu'elle est fortement sélective vis-à-vis du glucose. On peut par exemple utiliser la zéolithe X sous forme potassium telle que décrite dans la publication "A liquide phase chromatographic study of sorption and diffusion of glucose and fructose in NAX and KX zeolite crystals", Ching et al., Zeolites, 1988, vol. 8, pages 68 à 73.

Après une durée de réaction de 45 min, correspondant à un temps supérieur au temps nécessaire à l'équilibre de la réaction d'isomérisation sans destruction des sucres (c'est-à-dire à la durée de réaction permettant d'obtenir une sélectivité de 100 %), on vidange le réacteur, on stoppe la réaction par refroidissement brutal, on filtre la solution et on lave successivement le solide à la même température. On analyse ensuite les teneurs en glucose et en fructose de la solution liquide de réaction et de la solution issue de la désorption de la zéolithe.

Avec une quantité d'eau totale initiale de 230 g, la solution de glucose de départ avait une concentration de 500 g par litre.

A la fin de la réaction (45 min), on retrouve les 170 g de sucre avec les proportions pondérales suivantes : 19 % de fructose, 80 % de glucose, et 1 % de mannose.

### EXEMPLE 2

On réalise le même essai que dans l'exemple 1 mais en plaçant le réacteur à 60° C.

On retrouve, à l'issue de l'essai, 95 % des 170 g de sucre avec les proportions pondérales suivantes : 2 % de fructose, 98 % de glucose et 0 % de mannose.

Ces exemples démontrent que la zéolithe XK utilisée procure une adsorption sélective du glucose à 60° C sans isomérisation, et une excellente isomérisation du glucose en fructose à 95° C.

Avec une telle zéolithe, on peut donc obtenir à la sortie 4 de l'installation représentée sur la figure, une solution pure de fructose, en continu, en une seule étape, et à un coût extrêmement faible.

## Revendications

1. Procédé de préparation en continu d'une solution de cétose -notamment de cétohexose- à partir d'une solution sucrée de départ comprenant au moins un aldose -notamment au moins un aldohexose-, caractérisé en ce qu'on utilise au moins un réacteur (1) multicontact continu vertical, en ce qu'on introduit en continu la solution sucrée de départ dans le réacteur (1), en ce qu'on fait circuler en continu dans le réacteur (1) au moins un tectosilicate ou une argile apte à former un catalyseur hétérogène d'isomérisation des aldoses en cétoses, et en ce qu'on fait circuler de haut en bas dans le réacteur (1) au moins un solide microporeux d'adsorption sélective choisi pour adsorber sélectivement soit les aldoses de la solution de départ, soit les cétoses issus de l'isomérisation, et être compatible avec les conditions d'isomérisation.

2. Procédé selon la revendication 1, caractérisé en ce que :
- on utilise un seul réacteur multicontact (1),
- on introduit la solution sucrée de départ à un niveau intermédiaire du réacteur (1),
- on choisit au moins un solide microporeux d'adsorption sélective des aldoses de la solution sucrée de départ,
- on introduit dans le réacteur (1) un courant continu d'un solvant de la solution sucrée de départ à un niveau du réacteur inférieur à celui auquel on introduit la solution sucrée de départ,
- on extrait en continu à contre-courant la solution de cétose à un niveau du réacteur (1) au-dessus de celui auquel on introduit la solution sucrée de départ, de sorte que le procédé peut être effectué en une seule étape dans un seul réacteur (1).

3. Procédé selon l'une des revendications 1 et 2, caractérisé en ce qu'on contrôle la température au sein du(des) réacteur(s) (1) selon au moins deux zones (14, 15) distinctes, dont la première (14) est maintenue à une température comprise entre 30° C et 90° C choisie pour favoriser l'adsorption par le solide microporeux, et dont la deuxième (15) est maintenue à une température comprise entre 90° C et 110° C choisie pour favoriser l'isomérisation des aldoses en cétoses.

4. Procédé selon la revendication 3, caractérisé en ce qu'on maintient une température de l'ordre de 60° C dans la première zone d'adsorption (14) et une température de l'ordre de 95° C à 100° C dans la deuxième zone d'isomérisation (15).

5. Procédé selon l'une des revendications 3 et 4, caractérisé en ce que la première zone d'adsorption (14) est située au-dessus de l'introduction (6) de la solution sucrée de départ dans le réacteur (1), et en ce que la deuxième zone d'isomérisation (15) est située en-dessous de l'introduction (6) de la solution sucrée de départ dans le réacteur (1).

6. Procédé selon l'une des revendications 1 à 5, caractérisé en ce qu'on utilise, à titre de catalyseur hétérogène d'isomérisation, un tectosilicate ou une argile sous forme non protonique fonctionnalisé par des cations métalliques -notamment alcalins ou alcalino-terreux-.

7. Procédé selon l'une des revendications 1 à 6, caractérisé en ce qu'on utilise un tectosilicate ou une argile à titre .de solide microporeux d'adsorption sélective.

8. Procédé selon l'une des revendications 1 à 7, caractérisé en ce qu'on utilise un solide microporeux d'adsorption sélective en morceaux préformés, notamment sous forme extrudée.

9. Procédé selon l'une des revendications 1 à 8, caractérisé en ce qu'on utilise un catalyseur distinct du solide microporeux d'adsorption sélective.

10. Procédé selon l'une des revendications 1 à 8, caractérisé en ce qu'on utilise un tectosilicate ou une argile faisant office de catalyseur d'isomérisation et de solide microporeux d'adsorption sélective.

11. Procédé selon la revendication 10, caractérisé en ce qu'on choisit le tectosilicate ou l'argile pour qu'il fasse office de catalyseur dans une plage de températures différente de celle où il fait office de solide microporeux d'adsorption sélective des aldoses.

12. Procédé selon l'une des revendications 10 et 11, caractérisé en ce qu'on utilise une zéolithe X sous forme potassium ou baryum.

13. Procédé selon l'une des revendications 1 à 12, caractérisé en ce qu'on utilise une colonne pulsée à titre de réacteur.

14. Installation de mise en oeuvre du procédé selon l'une des revendications 1 à 13, caractérisée en ce qu'elle comporte un réacteur continu vertical multicontact (1), notamment une colonne pulsée, et des moyens (12, 13) de contrôle de la température régnant au sein du réacteur (1) selon au moins deux zones distinctes (14, 15) correspondant respectivement à au moins deux températures distinctes.

15. Installation selon la revendication 13, caractérisée en ce qu'elle comporte une alimentation (6) en solution de départ située à un niveau intermédiaire du réacteur (1), et en ce que les moyens (12, 13) de contrôle sont adaptés pour contrôler indépendamment la température au-dessus et en dessous de l'alimentation (6) de solution de départ.

## Patentansprüche

1. Präparation im Dauerbetrieb einer Ketoselösung - insbesondere einer Ketohexoselösung - ab einer ursprünglichen Zuckerlösung mit mindestens einer Aldose - insbesondere mindestens einer Aldohexose -, dadurch gekennzeichnet, dass man mindestens einen vertikalen kontinuierlichen Mehrkontakt-Reaktor (1) verwendet, dass man die ursprüngliche Zuckerlösung kontinuierlich in den Reaktor (1) eingibt, dass man mindestens ein Tektosilikat oder einen Ton kontinuierlich im Reaktor (1) umlaufen lässt, der geeignet ist, einen heterogenen Isomerisierungskatalysator der Aldosen in Ketosen zu bilden, und dass man mindestens einen mikroporösen, selektiven Adsorptionsfestkörper von oben nach unten im Reaktor (1) umlaufen lässt, der gewählt wurde, um entweder die Aldosen der Ausgangslösung oder die Ketosen aus der Isomerisierung selektiv zu adsorbieren und der mit den Isomerisierungsbedingungen kompatibel ist.

2. Verfahren nach Anspruch 1, dadurch gekennzeichnet, dass :
- man einen einzigen Mehrkontakt-Reaktor (1) verwendet,
- man die ursprüngliche Zuckerlösung in eine Zwischenstufe des Reaktors (1) eingibt,
- man mindestens einen mikroporösen, selektiven Adsorptionsfestkörper der Aldosen der ursprünglichen Zuckerlösung wählt,
- man in den Reaktor (1) einen Gleichstrom eines Lösemittels der ursprünglichen Zuckerlösung in einer Stufe des Reaktors einführt, die unter derjenigen liegt, in der man die ursprüngliche Zuckerlösung eingibt,
- man im Dauerbetrieb mit Gegenstrom die Ketoselösung in einer Stufe des Reaktors (1) entnimmt, die über derjenigen liegt, in die man die ursprüngliche Zuckerlösung einführt, so dass das Verfahren in einer einzigen Etappe in einem einzigen Reaktor (1) durchgeführt werden kann.

3. Verfahren nach einem der Ansprüche 1 und 2, dadurch gekennzeichnet, dass man die Temperatur innerhalb des Reaktors (der Reaktoren) (1) nach mindestens zwei verschiedenen Zonen (14, 15) prüft, wovon die erste (14) auf eine für die Begünstigung der Adsorption durch den mikroporösen Festkörper gewählten Temperatur von 30°C bis 90°C gehalten wird, und wovon die zweite (15) auf eine für die Begünstigung der Isomerisierung der Aldosen in Ketosen gewählten Temperatur von 90°C bis 110°C gehalten wird.

4. Verfahren nach Anspruch 3, dadurch gekennzeichnet, dass man in der ersten Adsorptionszone (14) eine Temperatur von etwa 60°C und in der zweiten Isomerisierungszone (15) eine Temperatur von etwa 95°C aufrechterhält.

5. Verfahren nach einem der Ansprüche 3 und 4, dadurch gekennzeichnet, dass die erste Adsorptionszone (14) über der Einführung (6) der ursprünglichen Zuckerlösung in den Reaktor (1) liegt, und dass die zweite Isomerisierungszone (15) unter der Einführung (6) der ursprünglichen Zuckerlösung in den Reaktor (1) liegt.

6. Verfahren nach einem der Ansprüche 1 bis 5, dadurch gekennzeichnet, dass man als heterogenen Isomerisierungskatalysator ein Tektosilikat oder einen Ton in nicht protonischer Form verwendet, der von Metallkationen - insbesondere alkalischen oder erdalkalischen - funktionalisiert wird.

7. Verfahren nach einem der Ansprüche 1 bis 6, dadurch gekennzeichnet, dass man ein Tektosilikat oder einen Ton als selektiven, mikroporösen Adsorptionsfestkörper verwendet.

8. Verfahren nach einem der Ansprüche 1 bis 7, dadurch gekennzeichnet, dass man einen selektiven, mikroporösen Adsorptionsfestkörper in vorgeformten Stücken, insbesondere in extrudierter Form, verwendet.

9. Verfahren nach einem der Ansprüche 1 bis 8, dadurch gekennzeichnet, dass man einen sich vom selektiven, mikroporösen Adsorptionsfestkörper unterscheidenden Katalysator verwendet.

10. Verfahren nach einem der Ansprüche 1 bis 8, dadurch gekennzeichnet, dass man ein Tektosilikat oder einen Ton verwendet, der als Isomerisierungskatalysator und als selektiver, mikroporöser Adsorptionsfestkörper dient.

11. Verfahren anch Anspruch 10, dadurch gekennzeichnet, dass man Tektosilikat oder Ton wählt, um als Katalysator in einem Temperaturbereich zu dienen, der sich von demjenigen unterscheidet, in dem er als selektiver, mikroporöser Adsorptionsfestkörper der Aldosen dient.

12. Verfahren nach einem der Ansprüche 10 und 11, dadurch gekennzeichnet, dass man einen Zeolithen X in Form von Kalium oder Barium verwendet.

13. Verfahren nach einem der Ansprüche 1 bis 12, dadurch gekennzeichnet, dass man als Reaktor eine Pulsationskolonne verwendet.

14. Anlage zur Umsetzung des Verfahrens nach einem der Ansprüche 1 bis 13, dadurch gekennzeichnet, dass sie einen vertikalen, kontinuierlichen Mehrkontakt-Reaktor (1) umfasst, insbesondere eine Pulsationskolonne, sowie Kontrollmittel (12, 13) der innerhalb des Reaktors (1) herrschenden Temperatur nach mindestens zwei verschiedenen Zonen (14, 15), die jeweils mindestens zwei verschiedenen Temperaturen entsprechen.

15. Anlage nach Anspruch 14, dadurch gekennzeichnet, dass sie eine Versorgung (6) mit einer Ausgangslösung umfasst, die auf einer Zwischenstufe des Reaktors (1) angeordnet ist, und dass die Kontrollmittel (12, 13) geeignet sind, um die Temperatur über und unter der Versorgung (6) mit Ausgangslösung unabhängig zu kontrollieren.

## Claims

1. A process for the continuous preparation of a solution of ketose, particularly ketohexose, from an initial sugar solution comprising at least one aldose, in particular at least one aldohexose, characterized by the use of at least one vertical continuous multi-contact reactor (1), by the fact that the initial sugar solution is introduced continuously into the reactor (1), that at least one tectosilicate or a clay suitable to form a heterogeneous catalyst for the isomerization of the aldoses and the ketoses is continuously circulated in the reactor (1), and that at least one microporous selective adsorption solid selected for the selective adsorption either of the aldoses from the initial solution or of the ketoses coming from the isomerization, while being compatible with the isomerization conditions, is circulated in downward direction in the reactor (1).

2. A process according to Claim 1, characterized by the fact that:
- a single multi-contact reactor (1) is used;
- the initial sugar solution is introduced at an intermediate level of the reactor (1);
- at least one microporous solid of selective adsorption of the aldoses from the initial sugar solution is selected;
- a continuous stream of the solvent of the initial sugar solution is introduced into the reactor (1) at a level of the reactor below that at which the initial sugar solution is introduced;
- the solution of ketose is continuously extracted countercurrent at a level of the reactor (1) located above the level at which the initial sugar solution is introduced, so that the process can be carried out in single step in a single reactor (1).

3. A process according to one of Claims 1 and 2, characterized by the fact that the temperature within the reactor or reactors (1) is controlled in at least two different zones (14, 15), the first of which (14) is maintained at a temperature of between 30°C and 90°C, selected so as to favor the adsorption by the microporous solid and the second of which (15) is maintained at a temperature of between 90°C and 110°C, selected so as to favor the isomerization of the aldoses into ketoses.

4. A process according to Claim 3, characterized by the fact that a temperature of about 60°C is maintained in the first adsorption zone (14) and a temperature of about 95°C to 100°C is maintained in the second isomerization zone (15).

5. A process according to one of Claims 3 and 4, characterized by the fact that the first adsorption zone (14) is located above the introduction (6) of the initial sugar solution into the reactor (1), and that the second isomerization zone (15) is located below the introduction (6) of the initial sugar solution into the reactor (1).

6. A process according to one of Claims 1 to 5, characterized by the fact that as heterogenous isomerization catalyst there is used a tectosilicate or a clay in non-protonic form, functionalized by metal cations, in particular alkaline or alkaline-earth cations.

7. A process according to one of Claims 1 to 6, characterized by the fact that a tectosilicate or a clay is used as microporous selective adsorption solid.

8. A process according to one of Claims 1 to 7, characterized by the fact that a selective adsorption microporous solid in preformed pieces, in particular in extruded form, is used.

9. A process according to one of Claims 1 to 8, characterized by the fact that a catalyst other than the selective microporous adsorption solid is used.

10. A process according to one of Claims 1 to 8, characterized by the fact that a tectosilicate or a clay serving as isomerization catalyst and as microporous selective adsorption solid is used.

11. A process according to Claim 10, characterized by the fact that the tectosilicate or clay is selected so that it serves as catalyst in a temperature range different from that in which it serves as selective microporous adsorption solid for the aldoses.

12. A process according to one of Claims 10 and 11, characterized by the fact that an X zeolite in potassium or barium form is used.

13. A process according to one of Claims 1 to 12, characterized by the fact that a pulsed column is used as reactor.

14. An installation for the carrying out of the process according to one of Claims 1 to 13, characterized by the fact that it comprises a multi-contact vertical continuous reactor (1), in particular a pulsed column, and means (12, 13) for controlling the temperature prevailing within the reactor (1) in at least two different zones (14, 15) corresponding to at least two different temperatures.

15. An installation according to Claim 13, characterized by the fact that it comprises an initial solution feed (6) located at an intermediate level of the reactor (1), and that the control means (12, 13) are adapted to control independently the temperature above and below the initial solution feed (6).
